(19) Europäisches Patentamt
European Patent Office
Office européen des brevets

(11) Publication number: 0 118 571 A1

# EUROPEAN PATENT APPLICATION

(21) Application number: 83102174.6

(22) Date of filing: 05.03.83

(51) Int. Cl.³: A 61 K 31/35

(43) Date of publication of application: 19.09.84 Bulletin 84/38

(84) Designated Contracting States: CH DE FR GB IT LI NL

(71) Applicant: Kaken Pharmaceutical Co., Ltd.
No. 28-8, Honkomagome 2-chome Bunkyo-ku
Tokyo(JP)

(72) Inventor: Okuda, Jun No. 3785-1632, Aza-Kuroishi
Oaza-Shimada Tenpaku-cho
Tenpaku-ku Nagoya-shi Aichi-ken(JP)

(72) Inventor: Miwa, Ichitomo
No. 1201, Miyukiyama Tenpaku-cho
Tenpaku-ku Nagoya-shi Aichi-ken(JP)

(72) Inventor: Inagaki, Kazuhiro
No. 55-57, Aza-Nishisaka Iwazu-cho
Okazaki-shi Aichi-ken(JP)

(72) Inventor: Horie, Tokunaru
No. 8-5, Kitayami-cho 4-chome
Tokushima-shi Tokushima-ken(JP)

(72) Inventor: Nakayama, Mitsuru
No. 1, Ushitahon-machi 6-chome Higashi-ku
Hiroshima-shi Hiroshima-ken(JP)

(74) Representative: Türk, Dietmar, Dr. rer. nat. et al,
Redies, Redies, Türk & Gille Patentanwälte
Brucknerstrasse 20
D-4000 Düsseldorf 13(DE)

(54) **Inhibitor of aldose reductase.**

(57) A inhibitor of aldose reductase which contains, as the effective ingredient, a flavone derivative having the following formula:

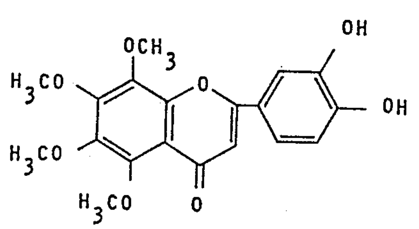

or a pharmaceutically acceptable salt thereof, and a pharmaceutically acceptable carrier. The inhibitor is useful for preventing, reducing and treating complications of diabetes.

EP 0 118 571 A1

# INHIBITOR OF ALDOSE REDUCTASE

The present invention relates to an inhibitor of aldose reductase (hereinafter referred to as "AR") containing a flavone derivative or a pharmaceutically acceptable salt thereof as an effective ingredient.

Complications of diabetes such as cataract and diseases of the retina and the kidney are caused by an unnecessary and harmful accumulation of the corresponding polyols derived from sugars by AR. For instance, sugar cataract is caused by turbidity of a lens which is caused by unnecessary and harmful accumulation of the corresponding sugar alcohols derived from glucose and galactose by AR which exists in a lens of an eyeball. Accordingly, it is essential to inhibit the enzyme activity of AR being a direct cause of the complications as strong as possible in order to effectively prevent, reduce or treat the complications.

There hitherto have been provided many compounds such as alrestatin and sorbinil as effective ingredients of inhibitors of AR. The most potent example of those inhibitors was quercitrin which is one of flavone derivatives having the following formula (I):

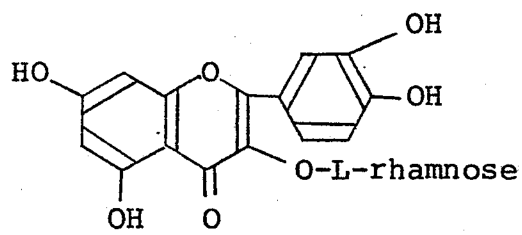

(I)

Quercitrin, however, is not potent enough to inhibit AR activity.

As a result of extensive study on dozens of flavone derivatives to find more potent inhibitors of AR by utilizing AR from rat and bovine lenses, it has now been found that 3',4'-dihydroxy-5,6,7,8-tetramethoxyflavone (hereinafter referred to as "LARI") and the salt thereof has a particularly potent inhibitory activity against AR.

In accordance with the present invention, there

can be provided a pharmaceutical composition having a particularly potent inhibitory activity against AR containing, as the effective ingredient, a flavone derivative having the following formula (II):

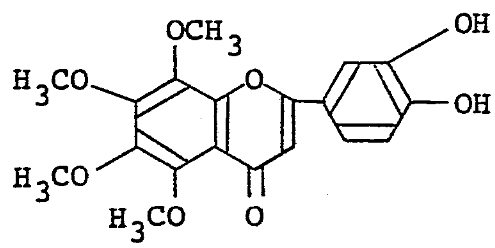

(II)

or a pharmaceutically acceptable salt of the flavone derivative and a pharmaceutically acceptable carrier.

Though all the dozens of flavone derivatives including the above-mentioned quercitrin obtained by chemical synthesis have a flavone skeleton, there is a large difference in inhibitory activities against AR of the above flavone derivatives depending on the kind and number of the binding groups such as hydroxyl group, methoxyl group or acetoxyl group, and the binding position.

The compound (II) or the pharmaceutically acceptable salt thereof is a typical example of inhibitors of AR having the most potent inhibitory activity against AR. LARI inhibits rat lens AR (hereinafter referred to as "RLAR") with a concentration of inhibitor needed to elicit 50 % inhibition (hereinafter referred to as "$IC_{50}$") value of $3.6 \times 10^{-8}$M and inhibits bovine lens AR (hereinafter referred to as "BLAR") with an $IC_{50}$ value of $1.8 \times 10^{-7}$M, while quercitrin inhibits RLAR and BLAR with $IC_{50}$ values of $4.9 \times 10^{-7}$M and $3.3 \times 10^{-6}$M, respectively. The inhibitory activity against AR of LARI is about 17 times as potent as that of quercitrin.

A process for preparing LARI being an effective ingredient of the inhibitor of the present invention can be summarized as follows: LARI can be prepared by hydrogenolysis of 3',4'-bis(benzyloxy)-5,6,7,8-tetramethoxyflavone prepared from 2-hydroxy-3,4,5,6-tetramethoxyacetophenone employed as a starting material.

The salt of LARI with Na, K, $NH_4$, Mg, and the like can be easily obtained in a usual manner.

LARI or the salt thereof has a highly potent inhibitory activity against AR and the inhibitor of the present invention containing LARI as an effective ingredient has an excellent effect on preventing, reducing or treating the complications of diabetes.

The inhibitor of the present invention can be formulated by a usual manner in a form of tablet, powder, syrup and liquid for injections or ophthalmic solution with conventional pharmaceutical carriers or media and administered both orally and parenterally. Examples of the carrier or medium are, for instance, excipients, binders, lubricants, coloring agents, perfumes, emulsifying agents, dispersing agents, and the like. If necessary, sterilized water, vegetable oil or harmless organic solvent can be used with the above carrier or medium.

Though the usual dosage is 100 mg. or less/kg. body weight/day as a flavone derivative to adult, the preferable dosage is determined by physicians doctors according to conditions of patients.

The present invention is more specifically described and explained by means of the following Examples. It is to be understood that the present invention is not limited to the Examples and various changes and modifications may be made in the invention without departing from the spirit and scope thereof.

Example 1

[Preparation of 3',4'-dihydroxy-5,6,7,8-tetramethoxyflavone (LARI)]

(1) Preparation of ω-[3,4-bis(benzyloxy)benzoyl]-2-hydroxy-3,4,5,6-tetramethoxyacetophenone

To 1.55 g. of 2-hydroxy-3,4,5,6-tetramethoxyacetophenone was added 2.82 g. of 3,4-bis(benzyloxy)benzoyl chloride and 10 ml. of pyridine and

the mixture was heated at 120°C. for 2 hours. The resulting reaction mixture was poured into a mixture of ice and hydrochloric acid and the precipitated oily material was extracted with ether. The ether phase was washed with a diluted hydrochloric acid, an aqueous sodium carbonate solution and water, and then dried. After distilling away ether, the resulting oily residue was dried in a desiccator to give a crude ester.

Into 15 ml. of pyridine was dissolved the obtained ester and added 4 g. of powdered pottasium hydroxide. After the resulting mixture was heated at 60°C. for 4 hours with stirring, the reaction mixture was poured into a mixture of water and hydrochloric acid and the precipitated oily material was extracted with ethyl acetate. The ethyl acetate phase was washed with a diluted hydrochloric acid, an aqueous sodium carbonate solution and water, and then dried.

The solvent was distilled away to give 3.16 g. of oily crude ω-[3,4-bis(benzyloxy)benzoyl]-2-hydroxy-3,4,5,6-tetramethoxyacetophenone.

(2) Preparation of 3',4'-bis(benzyloxy)-5,6,7,8-tetramethoxyflavone

Into 15 to 20 ml. of acetic acid was dissolved 2.90 g. of crude ω-[3,4-bis(benzyloxy)benzoyl]-2-hydroxy-3,4,5,6-tetramethoxyacetophenone obtained in Example 1 and added 3 g. of anhydrous sodium acetate. The resulting mixture was refluxed for 4 hours and added water for separating the precipitate. Recrystallization from ethyl acetate yielded 1.46 g. (44 % of the theoretical amount) of the desired compound being a colorless needle crystal.

mp: 157 to 157.5°C

Elementary analysis for $C_{33}H_{30}O_8$ (MW: 554.60)

Calcd. (%): C 71.47, H 5.45

Found (%): C 71.33, H 5.27

(3) Preparation of 3',4'-dihydroxy-5,6,7,8-tetramethoxyflavone (LARI)

Into 200 ml. of a mixed solvent of ethyl

acetate and methanol at a ratio of 1:1 was dissolved 1.11 g. of 3',4'-bis(benzyloxy)-5,6,7,8-tetramethoxyflavone and added 0.2 to 0.3 g. of 10 % palladium-charcoal. The resulting mixture was mixed with shaking in a stream of $H_2$ gas until $H_2$ absorption was completed. After filtering out the catalyst, the obtained filtrate was concentrated. The residue was recrystallized from methanol to give 0.74 g. (97 % of the theoretical amount) of the desired compound being a colorless prismatic crystal.

mp: 223 to 225$^{o}$C. (decompose)

Elementary analysis for $C_{19}H_{18}O_8$ (MW: 374.35)

Calcd. (%): C 60.96, H 4.85

Found (%): C 61.18, H 4.78

## Recipe Example

The inhibitory activitiy against AR of LARI was determined according to the following method. As reference examples, there were employed quercitrin and two flavone derivatives having a relatively similar structure to that of LARI, i.e. 6,4'-dihydroxy-5,7,8,3'-tetramethoxyflavone and 5,4'-dihydroxy-6,7,8,3'-tetramethoxyflavone (hereinafter referred to as "Ref-1" and "Ref-2", respectively).

In Recipe Example, the followings were used. NADPH (reduced form of nicotinamide adenine dinucleotide phosphate) was purchased from the Oriental Yeast Co., Ltd. DL-Glyceraldehyde and quercitrin were purchased from Nakarai Chemicals, Ltd. DEAE-Sephacel and Sephadex G-75 were purchased from Pharmacia Fine Chemicals. Matrex gel red A was purchased from the Amicon Co. All other chemicals were of the highest grade commercially available.

(1) Materials

Rat lenses were removed from eyes of rats of the Wistar strain weighing 200 to 250 g. Bovine eyes were obtained from a local abattoir, and the lenses were removed and frozen until needed.

(2) Preparation of Inhibitor solution

All compounds to be subjected to examination were dissolved in propylene glycol being a useful solvent of low toxicity for some water-insoluble drugs. A $10^{-3}$M solution was prepared and diluted to the desired concentration with propylene glycol. To the reaction mixture were added aliquots of the diluted solution to yield concentrations of $10^{-8}$M to $10^{-5}$M and a propylene glycol concentration of 1 %.

(3) Assay of AR activity

AR assays were conducted according to the procedure of Hayman and Kinoshita (J. Biol. Chem. 240, p. 877 (1965)), except for the addition of ammonium sulfate instead of lithium sulfate to the reaction mixture. Assays were performed at 30°C. in 0.1 M sodium phosphate buffer (pH 6.2) containing 0.4 M ammonium sulfate, 10 mM DL-glyceraldehyde and 0.16 mM NADPH and the enzyme (0.010 to 0.016 unit) in a total volume of 1.0 ml. The effects of inhibitors on the enzyme activity were determined in the reaction mixture containing 10 µl. of each inhibitor solution at the desired concentration. The blank reference to correct for non-specific reduction of NADPH contained all of the above compounds except the the above DL-glyceraldehyde. The reaction was initiated by the addition of the above DL-glyceraldehyde, and the rate of NADPH oxidation was followed by recording the decrease in absorbance at 340 nm. on a Gilford model 250 spectrophotometer.

(4) Purification of lens AR

BLAR and RLAR were purified according to the method of Inagaki et al (Archs. Biochem. Biophys. 216, p. 337 (1982)). Briefly, a 40 to 75 % ammonium sulfate fraction was subjected to chromatography on DEAE-Sephacel, followed by two column chromatographic steps, i.e. affinity chromatography using Mātrex gel red A and gel filtration on Sephadex G-75. In the procedure, a key step was affinity chromatography on Mātrex gel red A. BLAR adsorbed on the gel was eluted with 0.33 mM

NADPH, elution of RLAR adsorbed on the gel was effected by means of a linear gradient of NaCl (0 to 1.0 M). BLAR and RLAR were purified over 12,000-fold (4.8 units/mg. protein) and 380-fold (4.7 units/mg. protein), respectively, and used for determining $IC_{50}$ values.

(5) Determination of $IC_{50}$

The concentration of inhibitor needed to elicit a 50 % inhibition ($IC_{50}$) was determined according to the method of Kador et al (Docum. Ophthal. Proc. Series 18, p. 117 (1979)).

The results are shown in Table 1 and Table 2.

Table 1

| Compound | Inhibition against enzyme activity (%) | | | | | |
|---|---|---|---|---|---|---|
| | RLAR | | | BLAR | | |
| | $10^{-5}$M | $10^{-6}$M | $10^{-7}$M | $10^{-5}$M | $10^{-6}$M | $10^{-7}$M |
| LARI | 97 | 93 | 78 | 100 | 90 | 37 |
| Ref-1 | 58 | 22 | 6 | 63 | 19 | 0 |
| Ref-2 | 40 | 15 | 0 | 56 | 19 | 0 |
| quercitrin | 84 | 56 | 38 | 66 | 32 | 0 |

Table 2

| Compound | $IC_{50}$ (M) X $10^6$ RLAR | BLAR |
|---|---|---|
| LARI | 0.030 | 0.18 |
| quercitrin | 0.49 | 3.3 |

It is clear from the results of Tables 1 and 2 that the flavone derivative being an effective ingredient of the inhibitor of the present invention has highly potent inhibitory activity against AR comparing with those of the other flavone derivatives including quercitrin.

WHAT WE CLAIM IS:

1. A pharmaceutical composition having an inhibitory activity against aldose reductase which comprises, as the effective ingredient, a flavone derivative having the following formula (II):

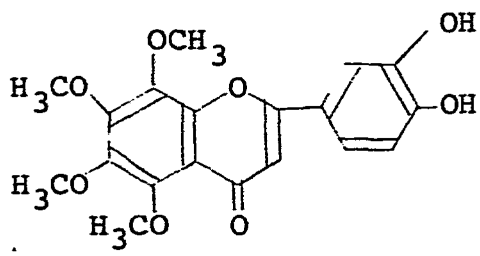

(II)

or a pharmaceutically acceptable salt of said flavone derivative, and a pharmaceutically acceptable carrier.

European Patent Office

# EUROPEAN SEARCH REPORT

0118571

Application number

EP 83 10 2174

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| X | NIPPON KAGAKU KAISHI, no. 1, 1983, pages 161-165 M. NAKAYAMA et al.: "Synthesis of 3',4',5-Trihydroxy-6,7,8-trimethoxyflavone and its related compounds" & CHEMICAL ABSTRACTS, vol. 98, 1983, page 492, no. 160487r * Pages 161,163,165 * ----- | 1 | A 61 K 31/35 |
| | | | TECHNICAL FIELDS SEARCHED (Int. Cl. 3) |
| | | | C 07 D 311/00<br>A 61 K 31/00 |

The present search report has been drawn up for all claims

| Place of search | Date of completion of the search | Examiner |
|---|---|---|
| THE HAGUE | 11-11-1983 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document